# EUROPEAN PATENT SPECIFICATION

(11) **EP 4 321 098 B1**
(45) Date of publication and mention of the grant of the patent: **07.10.2026**
(21) Application number: 21936100.3
(22) Date of filing: 27.12.2021
(51) Int. Cl.: A61B 6/00, A61B 6/12

(54) **X-RAY IMAGING APPARATUS AND IMAGE PROCESSING PROGRAM**
RÖNTGENBBILDGEBUNGSVORRICHTUNG UND BILDVERARBEITUNGSPROGRAMM
DISPOSITIF D'IMAGERIE À RAYONS-X ET PROGRAMME DE TRAITEMENT D'IMAGES

(30) Priority: 07.04.2021 JP 2021065057
(43) Date of publication of application: 14.02.2024
(73) Proprietor: Shimadzu Corporation, Nakagyo-ku, Kyoto-shi, Kyoto 604-8511 (JP)
(72) Inventor: HU, Erzhong, Kyoto-shi, Kyoto 604-8511 (JP); HOSOMI, Naomasa, Kyoto-shi, Kyoto 604-8511 (JP)
(74) Representative: Müller-Boré & Partner Patentanwälte PartG mbB
(86) International application number: PCT/JP2021/048529
(87) International publication number: WO 2022/215303

(56) References cited:
- WO-A1-2019/138438
- WO-A1-2019/229119
- JP-A- 2016 034 451
- JP-A- 2020 036 773
- JP-A- 2021 013 685
- JP-A- 2021 049 111
- US-A1- 2021 015 440
- US-A1- 2021 085 267

## Description

### Technical Field

The present invention relates to an X-ray imaging apparatus and an image processing program, and more particularly to an X-ray imaging apparatus and an image processing program for checking the presence or absence of a target substance in a body of a subject.

### Background Art

Conventionally, there is a known a radiographic imaging system (X-ray imaging apparatus) for checking the presence or absence of hemostatic gauze (target object) in a body of a subject after an abdominal surgical operation. Such an apparatus is disclosed, for example, in Japanese Unexamined Patent Application Publication No. 2019-180605.

The radiographic imaging system described in the above-described Japanese Unexamined Patent Application Publication No. 2019-180605 performs processing of a radiographic image according to predefined processing procedures according to the inspection purpose. In this radiographic imaging system, for example, in the case where processing procedures have been set to check the presence or absence of hemostatic gauze after an abdominal surgical operation, foreign object enhancement processing is performed on the captured image. Further, in this radiographic imaging system, an abdominal image in which foreign object enhancement processing has been performed is displayed so that a foreign object (gauze) in a body of a subject can be easily recognized.

US 2021/015440 A1 describes a radiographic image processing apparatus comprising a detection unit including a discriminator that has been trained so as to discriminate a surgical tool region in an input radiographic image, on the basis of training data which consists of a composite image acquired by combining a first radiographic image including a human body and a second radiographic image including a surgical tool and correct answer data indicating the surgical tool region in the composite image.

US 2021/085267 A1 describes a radiographic image processing apparatus, a radiographic image processing method, and a radiographic image processing program which can check whether or not a surgical tool detection process is operating correctly.

### Prior Art Documents

### Patent Document

**Patent Document 1:** Japanese Unexamined Patent Application Publication No. No. 2019-180605

### SUMMARY OF THE INVENTION

### Problems to be Solved by the Invention

Here, although not specifically described in the above-described Patent Document 1, in the case of performing foreign object enhancement processing on an X-ray image showing a subject after a surgical operation in order for a doctor, etc., to check the presence or absence of a foreign object (such as, e.g., hemostatic gauze) in a body of a subject after the operation, as in the radiographic imaging system described in the above-described Patent Document 1, in some cases, edge enhancement processing is performed as foreign object enhancement processing. However, in the case of performing edge enhancement processing as foreign object enhancement processing, an enhanced image in which not only a foreign object but also a human body structure, such as, e.g., a bone of a subject, are emphasized is generated. In this case, the visibility of the foreign object in the enhanced image deteriorates, which makes it difficult to recognize the foreign object (target object) in the X-ray image.

The present invention has been made to solve the above-described problems, and one object of the present invention is to provide an X-ray imaging apparatus and an image processing program capable of easily checking the presence or absence of a target object included in an X-ray image when enhancing the target object included in the X-ray image showing a subject. The present invention is defined by the independent claims. Preferred examples are defined in the dependent claims.

### Effects of the Invention

In the X-ray imaging apparatus described below, the image processing apparatus described below, and the image processing program described below, at least one of an output image of the trained model in which the position of the target object is emphasized and a composite image generated so that the position of the target object is emphasized based on the output image and the X-ray image is generated as an enhanced image, based on the trained model that detects a region of a target object in the body of the subject in the X-ray image when the X-ray image is input. Then, the X-ray image and at least one of the output image and the composite image are caused to be displayed on the display unit simultaneously or switchably. With this, at least one of the output images and the composite image is generated as an enhanced image in which the position image of the target object is emphasized, based on the trained model that directly detects the region of the target object. Therefore, unlike the case in which an enhanced image in which both the target object and the human body structure, such as, e.g., a bone of the subject, are emphasized by edge enhancement processing, it is possible to suppress that the human body structure, such as, e.g., a bone of the subject, is emphasized in the enhanced image. As a result, in the case of emphasizing the target object included in the X-ray image showing the subject, the target object included in the X-ray image can be easily identified. Further, the X-ray image and at least one of the output images and the composite image as an enhanced image are displayed on the display unit simultaneously or switchably. Therefore, the target object included in the X-ray image can be easily confirmed by comparing the enhanced image with the X-ray image. Further, the final determination of the presence or absence of the target object in the body of the subject by a doctor, etc., is performed based on the X-ray image. Therefore, it is very effective that the target object included in the X-ray image can be easily confirmed by comparing the X-ray image with the enhanced image.

Here, in the case of emphasizing the target object in the X-ray image showing the subject by performing image processing using a trained model, it is conceivable to generate a trained model that removes the target object from the X-ray image and generate a removed image in which the target object has been removed from the X-ray image using the generated trained model to thereby generate an enhanced image by the difference between the X-ray image and the removed image. However, in the case where an enhanced image is generated by the difference between the X-ray image and the removed image, there is a case in which not only the target object but also the structure of the subject similar to the target object are emphasized in the enhanced image due to the removal of the structure of the subject (such as pelvis and femur) that are similar to the target object in the removed image. Therefore, in the case of performing image processing using a trained model that removes the target object from the X-ray image, the visibility of the target object in the enhanced image deteriorates, making it difficult to identify the target object in the X-ray image.

In the X-ray imaging apparatus described below, the image processing apparatus described below, and the image processing program described below, at least one of an output image of the trained model in which the position of the target object is emphasized and a composite image generated so that the position of the target object is emphasized based on the output image and the X-ray image is generated as an enhanced image, based on the trained model that detects a region of a target object in the body of the subject in the X-ray image when the X-ray image is input. Then, the X-ray image and at least one of the output image and the composite image are caused to be displayed on the display unit simultaneously or switchably. With this, at least one of the output image and the composite image is generated as an enhanced image in which the position image of the target object is emphasized, based on the trained model that directly detects the region of the target object. Therefore, unlike in the case in which a removed image is generated by a trained model that removes the target object from the X-ray image, and an enhanced image is generated by the difference between the X-ray image and the removed image, it is possible to suppress that the structure of the subject similar to the target object is emphasized in the enhanced image. As a result, even in the case of emphasizing the target object in the X-ray image showing the subject by performing image processing using a learned model, the target object in the X-ray image can be easily confirmed.

### BRIEF DESCRIPTION OF THE DRAWINGS

FIG. 1 is a diagram for describing a configuration of an X-ray imaging apparatus according to one embodiment.
FIG. 2 is a block diagram for describing a configuration of an X-ray imaging apparatus according to one embodiment.
FIG. 3 is a diagram showing one example of an X-ray image of a subject in which a target object is present in a body according to one embodiment.
FIG. 4 is a diagram for describing image processing using a trained model according to one embodiment.
FIG. 5 is a diagram for describing an output layer image according to one embodiment.
FIG. 6 is a diagram for describing an intermediate layer image according to one embodiment.
FIG. 7 is a diagram for describing generation of a trained model according to one embodiment.
FIG. 8 is a diagram for describing a colored image according to one embodiment.
FIG. 9 is a diagram for describing a colored superimposed image according to one embodiment.
FIG. 10 is a diagram for describing an intermediate layer superimposed image according to one embodiment.
FIG. 11 is a diagram for describing a display of a display unit according to one embodiment.
FIG. 12 is a flowchart for describing an image processing method according to one embodiment.

### EMBODIMENTS FOR CARRYING OUT THE INVENTION

Hereinafter, embodiments in which the present invention is embodied will be described based on the attached drawings.

### (Overall Configuration of X-ray Imaging Apparatus)

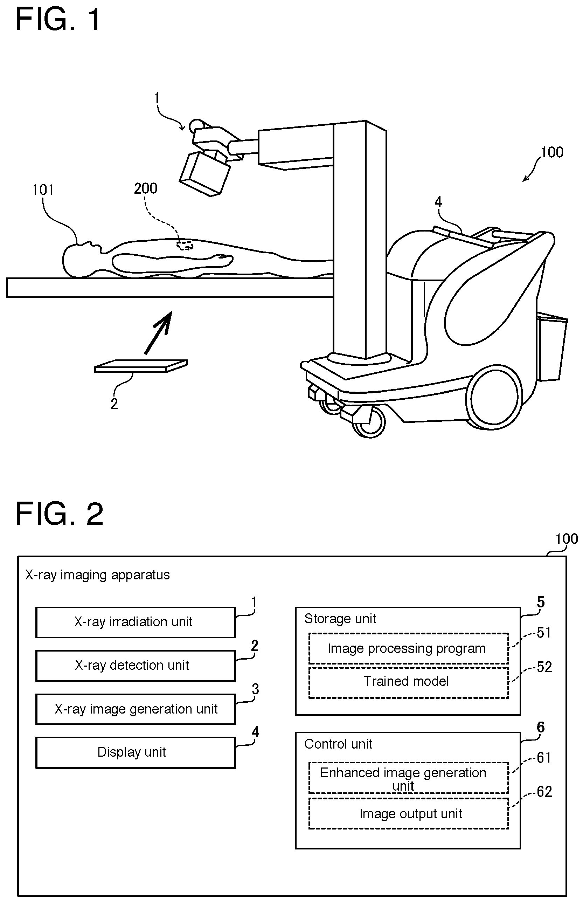

Referring to FIG. 1 to FIG. 11, an X-ray imaging apparatus 100 according to one embodiment of the present invention will be described.

As shown in FIG. 1, the X-ray imaging apparatus 100 performs X-ray imaging to identify a target object 200 in a body of a subject 101. For example, the X-ray imaging apparatus 100 performs X-ray imaging to check whether a target object 200 (retained foreign object) is left behind in the body of the subject 101 to whom an abdominal surgical operation has been performed in an operation room. The X-ray imaging apparatus 100 is, for example, an X-ray imaging apparatus for rounds configured to be entirely movable. The target object 200 is, for example, surgical operation gauze, suture needles, and forceps (e.g., hemostatic forceps).

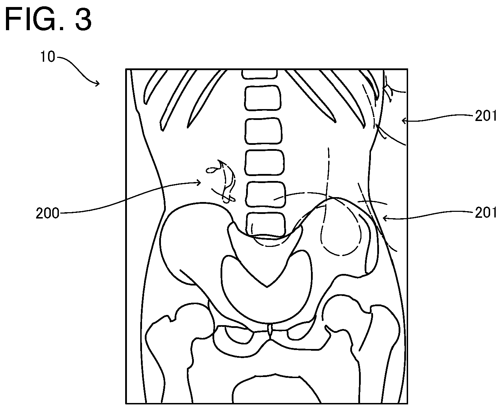

In general, when a surgical operation, such as, e.g., an abdominal surgical operation, has been performed, an operator, such as, e.g., a doctor, performs X-ray imaging on the subject 101 to confirm that no target object 200, such as, e.g., surgical operation gauze, suture needles, and forceps, is left behind (remains) in the body of the subject 101 after the closure. An operator, such as, e.g., a doctor, visually confirms the X-ray image 10 (see FIG. 3) of the subject 101 to check whether the target object 200 is left behind in the body of the subject 101.

### (X-ray Imaging Apparatus)

As shown in FIG. 2, the X-ray imaging apparatus 100 is provided with an X-ray irradiation unit 1, an X-ray detection unit 2, an X-ray image generation unit 3, a display unit 4, a storage unit 5, and a control unit 6. Note that the control unit 6 is one example of the "image processing apparatus" and the "computer" recited in original claims.

The X-ray irradiation unit 1 emits X-rays to the subject 101 after surgery. The X-ray irradiation unit 1 includes an X-ray tube that emits X-rays when a voltage is applied.

The X-ray detection unit 2 detects X-rays transmitted through the subject 101. The X-ray detection unit 2 outputs a detection signal based on the detected X-rays. The X-ray detection unit 2 includes, for example, an FPD (Flat Panel Detector). Further, the X-ray detection unit 2 is configured as a wireless type X-ray detector and outputs a detection signal as a wireless signal. Specifically, the X-ray detection unit 2 is configured to be connected to the X-ray image generation unit 3 in a communicable manner by a wireless connection using a wireless LAN or the like and output a detection signal as a wireless signal to the X-ray image generation unit 3.

As shown in FIG. 3, the X-ray image generation unit 3 controls the X-ray irradiation unit 1 and the X-ray detection unit 2 to control X-ray imaging. The X-ray image generation unit 3 generates an X-ray image 10 based on a detection signal of X-rays detected by the X-ray detection unit 2. The X-ray image generation unit 3 is configured to be communicable with the X-ray detection unit 2 by a wireless connection using a wireless LAN, etc. The X-ray image generation unit 3 includes a processor, such as, e.g., an FPGA (fieldprogrammable gate array). The X-ray image generation unit 3 outputs a generated X-ray image 10 to the control unit 6.

The X-ray image 10 shown in FIG. 3 is an image obtained by X-ray imaging the abdomen of the subject 101 after surgery. In the X-ray image 10 shown in FIG. 3, surgical gauze is included as the target object 200. Surgical operation gauze is woven with contrast threads that are less likely to transmit X-rays so that they are visible in the X-ray image 10 obtained by X-ray imaging after a surgical operation. Further, in the X-ray image 10 shown in FIG. 3, surgical wires and surgical clips are included as artificial structures 201 other than the target object 200.

The display unit 4 includes, for example, a touchscreen liquid crystal display. The display unit 4 displays various images, such as, e.g., an X-ray image 10. Further, the display unit 4 is configured to receive an input operation for operating the X-ray imaging apparatus 100 by an operator, such as, e.g., a doctor, based on the operation to the touch panel.

The storage unit 5 is configured by a storage unit, such as, e.g., a hard disk drive. The storage unit 5 stores image data, such as, e.g., the X-ray image 10. Further, the storage unit 5 stores various set values for operating the X-ray imaging apparatus 100. Further, the storage unit 5 stores programs used for the processing control of the X-ray imaging apparatus 100 by the control unit 6. Further, the storage unit 5 stores an image processing program 51. The image processing program 51 can be stored in the storage unit 5, for example, by reading it from a non-transitory portable storage medium, such as, e.g., an optical disk and a USB memory, or by downloading it via a network. Further, the storage unit 5 stores a trained model 52, which will be described later.

The control unit 6 is a computer configured to include, for example, a CPU (Central Processing Unit), a GPU (Graphics Processing Unit), a ROM (Read Only Memory), and a RAM (Random Access Memory). The control unit 6 includes, as functional configurations, an enhanced image generation unit 61 and an image output unit 62. In other words, the control unit 6 executes the image processing program 51 to function as the enhanced image generation unit 61 and the image output unit 62. Further, the enhanced image generation unit 61 and the image output unit 62 are functional blocks as software in the control unit 6, and are configured to function based on a command signal from the control unit 6 as hardware.

### (Image Processing)

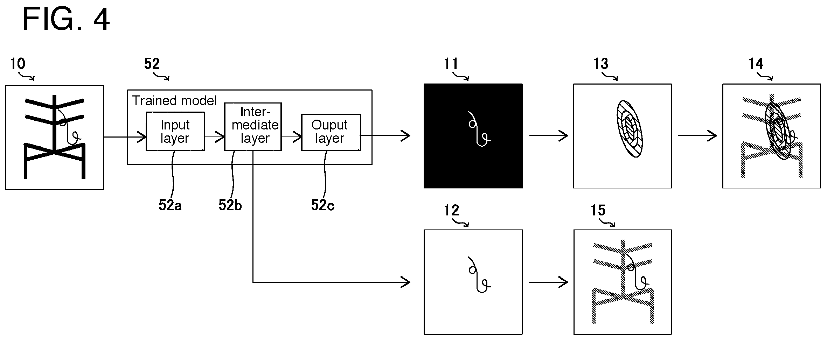

Here, in this embodiment, as shown in FIG. 4, the enhanced image generation unit 61 (control unit 6) generates, as enhanced images, based on the trained model 52 that detects the region of the target object 200 of the subject 101 in the X-ray image 10 when the X-ray image 10 generated by the X-ray image generation unit 3 is input, an output image (11, 12) of the trained model 52 in which the position of the target object 200 is emphasized and a composite image (14, 15) generated so that the position of the target object 200 is emphasized based on the output image (11, 12) and the X-ray image 10. Then, the image output unit 62 (control unit 6) makes the display unit 4 display the X-ray image 10 and at least one of the output image (11, 12) and the composite image (14, 15) generated by the enhanced image generation unit 61, simultaneously or switchably.

### (Generation of Output Image)

In this embodiment, as shown in FIG. 4 to FIG. 6, the enhanced image generation unit 61 (control unit 6) generates, as output images of the trained model 52, the output layer image 11 and the intermediate layer image 12, based on the trained model 52 generated by machine learning. The trained model 52 is generated by machine learning using deep learning. The trained model 52 is generated, for example, based on a U-Net, which is one type of a fully convolution network (FCN). The trained model 52 is generated by training to execute an image transformation (image reconstruction) that detects a portion estimated to be the target object 200 from the X-ray image 10 by transforming a pixel estimated to be the target object 200 out of each pixel of the X-ray image 10 that is the input. Note that the output layer image 11 and the intermediate layer image 12 are examples of the "enhanced image" and the "output image" recited in claims.

Further, the trained model 52 includes an input layer 52a, an intermediate layer 52b, and an output layer 52c. The input layer 52a receives an input image (X-ray image 10). The intermediate layer 52b acquires feature components (target object 200) from the input image received by the input layer 52a. Specifically, the intermediate layer 52b has a downsampling unit that acquires feature components (target object 200) from the input image while reducing the size of the input image, and an up-sampling unit that restores an image including the feature components reduced in size by the down-sampling unit to the original size (size of the input image). The intermediate layer 52b outputs the intermediate layer image 12 in which the target object 200 in the X-ray image 10 is emphasized. The output layer 52c has a sigmoid function as an activation function. The output layer 52c executes processing by a sigmoid function on the intermediate layer image 12 output from the intermediate layer 52b to generate and output the output layer image 11 showing the region of the target object 200 in the X-ray image 10. The output layer image 11 is configured to represent the probability that the pixel value of each pixel belongs to the region of the target object 200. In the output layer image 11, a pixel with a high probability of belonging to the region of the target object 200 (a pixel having a pixel value of 1 or close to 1) is displayed in white, and a pixel with a low probability of belonging to the region of the target object 200 (a pixel having a pixel value of 0 or close to 0) is displayed in black.

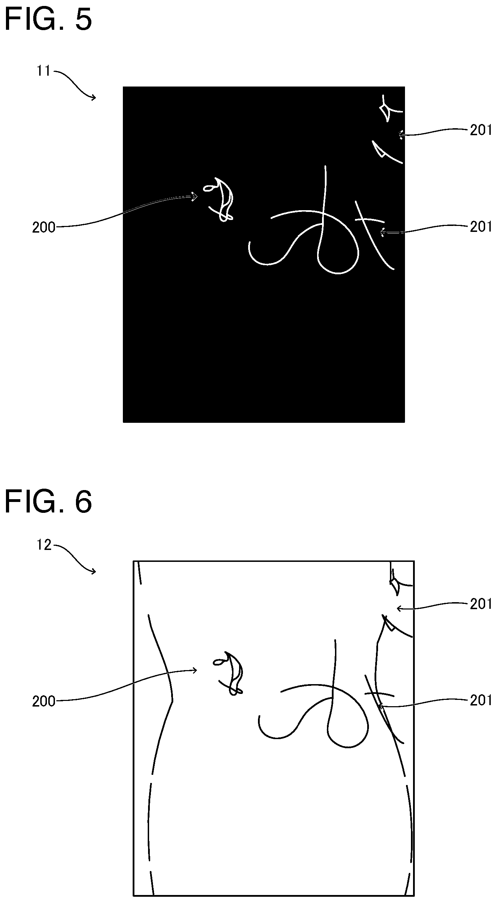

The output layer image 11 shown in FIG. 5 is an image acquired from the X-ray image 10 shown in FIG. 3 based on the trained model 52. In the output layer image 11 shown in FIG. 5, the structures of the subject 101, such as, e.g., a bone, have been removed to emphasize the target object 200. Further, in the output layer image 11 shown in FIG. 5, although the artificial structure 201, which is similar in shape (feature) to the target object 200, is also emphasized, an operator, such as, e.g., a doctor, can easily distinguish it from the target object 200 because he or she knows the position, the shape, etc., of the artificial structure 201.

The intermediate layer image 12 shown in FIG. 6 is an image acquired from the X-ray image 10 shown in FIG. 3 based on the trained model 52. In the output layer image 12 shown in FIG. 6, the structures of the subject 101, such as, e.g., bones, remain slightly, but the target object 200 is emphasized. Note that the remaining structures of the subject 101, such as, e.g., bones, are removed by the processing by a sigmoid function in the output layer 52c. Further, in the output layer image 12 shown in FIG. 6, although the artificial structure 201, which is similar in shape (feature) to the target object 200, is also emphasized, an operator, such as, e.g., a doctor, can easily distinguish it from the target object 200 because he or she recognizes the position, the shape, etc., of the artificial structure 201.

### (Generation of Trained Model)

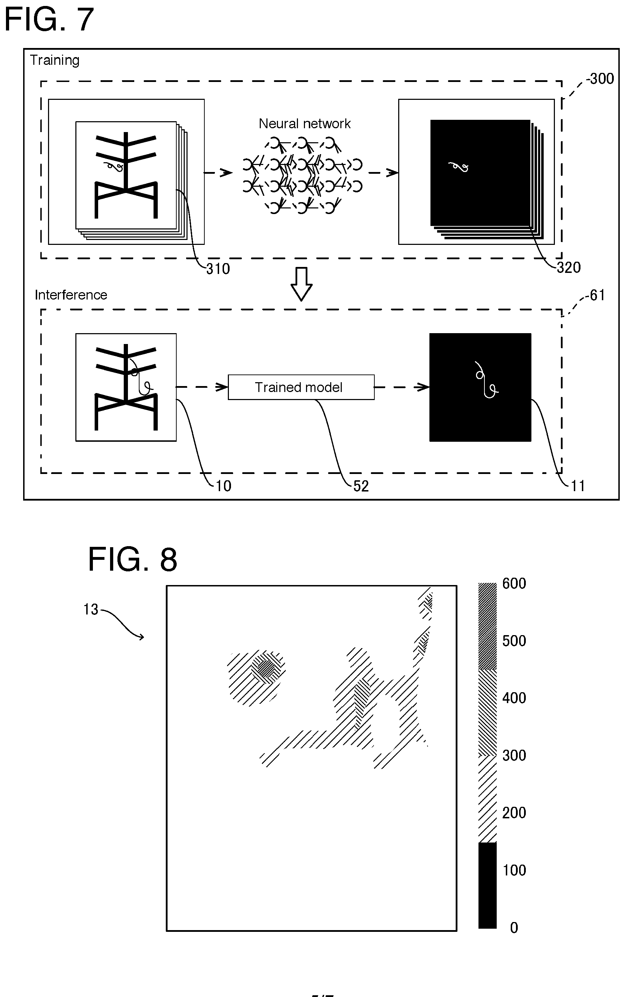

As shown in FIG. 7, in this embodiment, the trained model 52 is generated by executing machine learning so as to detect the target object 200, such as, e.g., surgical operation gauze, suture needles, and forceps, from the X-ray image 10. The trained model 52 is generated in advance by a training device 300 provided separately from the X-ray imaging apparatus 100. The training device 300 is a computer composed of, for example, a CPU, a GPU, a ROM, and a RAM. The training device 300 generates a trained model 52 by performing machine learning using deep learning, using a plurality of training input X-ray images 310 and a plurality of training output images 320 as training data (training set).

The training input X-ray image 310 is generated so as to simulate the X-ray image 10 showing the subject 101 in which the target object 200 is left behind in the body. The training output image 320 is generated so as to simulate that the target object 200 is detected from the training input X-ray image 310. The training input X-ray image 310 and the training output image 320 are generated so that the conditions (e.g., size) are similar to those of the X-ray image 10 used as the input in inference using the trained model 52.

### (Generation of Superimposed Image)

In this embodiment, as shown in FIG. 4, FIG. 8, and FIG. 9, the enhanced image generation unit 61 (control unit 6) generates, as a composite image, a colored superimposed image 14 in which the colored image 13 generated based on the output layer image 11 is superimposed on the X-ray image 10, based on the X-ray image 10 and the output layer image 11 of the trained model 52. Specifically, the enhanced image generation unit 61 colors the portion in the output layer image 11 corresponding to the target object 200 based on the output layer image 11 to thereby generate a colored image 13. Then, the enhanced image generation unit 61 generates the colored superimposed image 14 in which the generated colored image 13 is superimposed on the X-ray image 10. Note that the colored image 13 is one example of the "image generated based on an output image" recited in claims. Further, the colored superimposed image 14 is one example of the "enhanced image," the "composite image," and the "superimposed image" recited in claims.

The enhanced image generation unit 61 identifies the linear structure (shape) of the target object 200 in the output layer image 11 based on the output layer image 11, acquires the density in the predetermined region including the site where the linear structure of the target object 200 is located, and generates the colored image 13 as a heat map image (color map image) colored to vary according to the density.

For example, the enhanced image generation unit 61 performs binary-coded processing on the generated output layer image 11. Then, the enhanced image generation unit 61 detects the density of the linear structure in the output layer image 11 subjected to the binary-coded processing to thereby identify the portion (pixel) including the linear structure. Specifically, the enhanced image generation unit 61 extracts the features from the output layer image 11 to identify the linear structure by performing pattern recognition on the binarized output layer image 11.

For example, the enhanced image generation unit 61 extracts higher-order local autocorrelation (HLAC: Higher-order Local AutoCorrelation) features as features. For example, the enhanced image generation unit 61 acquires one pixel of the output layer image 11 as a reference point. And, the enhanced image generation unit 61 extracts features by local autocorrelation features in the predetermined region including (centered on) the reference point. The enhanced image generation unit 61 then measures the degree of agreement between the extracted features and the features of the linear structure (shape) set in advance to thereby identify (detect) the linear structure in the predetermined region including the reference point. The enhanced image generation unit 61 acquires the detected value of the linear structure in the predetermined region including the reference point, as the density of the linear structure in the predetermined region at the reference point. The enhanced image generation unit 61 acquires the local autocorrelation features as a reference point for each of the pixels in the output layer image 11 to thereby acquire the density (detection value) of the linear structure in each of the pixels in the output layer image 11. Here, the size of the predetermined region including the reference point may be, for example, a 3 x 3 pixel region, or a region larger than 3 x 3 pixels, such as, e.g., 9 x 9 pixels.

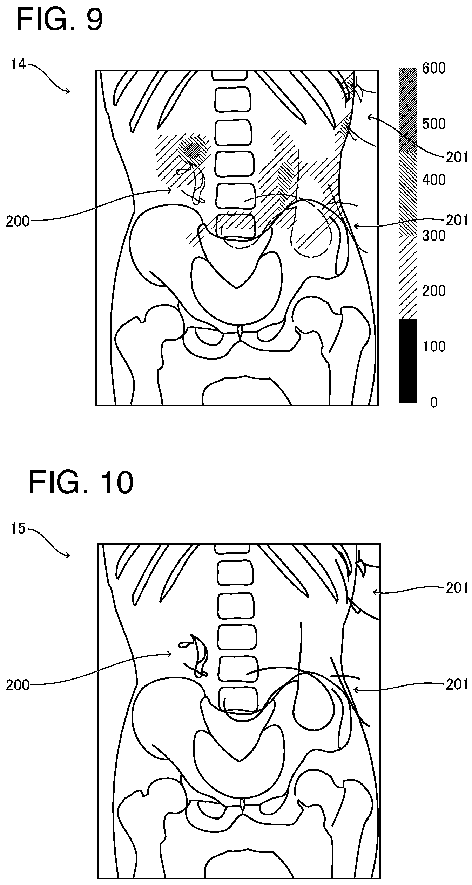

The enhanced image generation unit 61 then colors each pixel based on the density (detection value) of the linear structure acquired for each pixel in the output layer image 11 to thereby generate the colored image 13. For example, the enhanced image generation unit 61 colors each pixel so that the hue varies depending on the density value of the linear structure to generate the colored image 13. For example, the colored image 13 is colored in the order of red, yellow, green, and blue, from the highest density value to the lowest. That is, when the density value is large, the pixel is colored red, and when the density value is small, the pixel is colored blue. The enhanced image generation unit 61 sets the colors in the colored image 13 by correlating the range of values between 0 and 600 of the density (detection value) of the acquired linear structure to each color. Note that in FIG. 8 and FIG. 9, the difference in color is represented by the difference in hatching.

The enhanced image generation unit 61 generates, as described above, the colored image 13 capable of identifying, by the colors displayed, the degree to which the features at each pixel (each region) correspond to the features of the linear structure corresponding to the target object 200. The identification (extraction) of the linear structure (shape) in the case of generating the colored image 13 may be performed by acquiring features for each predetermined region by pattern recognition using an extraction method of features other than higher-order local autocorrelation features and identifying (detecting) the density (corresponding degree of the pattern) of the linear structure (shape) to color. In the above description, although an example of identifying surgical operation gauze as the target object 200 is described, also in the case where the target object 200 is a suture needle or forceps, etc., in the same manner, the colored image 13 is generated by identifying the linear structure (shape) from the output layer image 11.

The colored image 13 shown in FIG. 8 is an image acquired based on the output layer image 11 shown in FIG. 5. In the colored image 13 shown in FIG. 8, the position of the target object 200 is emphasized by being colored based on the linear structure of the target object 200. In the colored image 13 shown in FIG. 8, although the position of the artificial structure 201, which is similar in shape (features) to the target object 200, is also emphasized by being colored, the position of the artificial structure 201 is colored with a color (such as green) that represents a lower pixel density than the position of the target object 200. Therefore, an operator, such as, e.g., a doctor, can easily distinguish it from the target object 200. Note that the artificial structure 201 is estimated to be lower in the probability of belonging to the region of the target object 200 than the target object 200 in the output layer image 11, so the pixel density of the artificial structure 201 is lower than that of the position of the target object 200. Further, the colored image 13 does not include information on the shape of the target object 200 and that of the artificial structure 201.

The colored superimposed image 14 shown in FIG. 9 is an image in which the colored image 13 shown in FIG. 8 is superimposed on the X-ray image 10 shown in FIG. 3. In the colored superimposed image 14 shown in FIG. 9, the colored image 13 is superimposed on the X-ray image 10 to emphasize the position of the target object 200. Further, in the colored superimposed image 14 shown in FIG. 9, although the position of the artificial structure 201, which is similar in shape (features) to the target object 200, is also emphasized by being superimposed by the colored image 13, the position of the artificial structure 201 is colored with a color (such as green) that represents a lower pixel density than that of the position of the target object 200. Therefore, an operator, such as, e.g., a doctor, can easily distinguish it from the target object 200. Further, an operator, such as, e.g., a doctor, knows the position, the shape, etc., of the artificial structure 201 and, therefore, can easily distinguish it from the target object 200.

As shown in FIG. 4 and FIG. 10, the enhanced image generation unit 61 (control unit 6) generates, as a composite image, the intermediate layer superimposed image 15 in which the intermediate layer image 12 is superimposed on the X-ray image 10, based on the X-ray image 10 and the intermediate layer image 12 of the trained model 52. For example, the enhanced image generation unit 61 (control unit 6) generates the intermediate layer superimposed image 15 by superimposing the intermediate layer image 12 subjected to transmission processing on the X-ray image 10. Note that the intermediate layer superimposed image 15 is an example of the "composite image" and the "superimposed image" recited in claims.

The intermediate layer superimposed image 15 shown in FIG. 10 is an image in which the intermediate layer image 12 shown in FIG. 6 is superimposed on the X-ray image 10 shown in FIG. 3. In the intermediate layer superimposed image 15 shown in FIG. 10, the intermediate layer image 12 is superimposed on the X-ray image 10 to emphasize the position and the shape of the target object 200. Although in the intermediate layer superimposed image 15 shown in FIG. 10, although the position and the shape of the artificial structure 201, whose shape (features) is similar to that of the target object 200, are also emphasized by being colored, an operator, such as, e.g., a doctor, knows the position, the shape, etc., of the artificial structure 201. Therefore, the target object 200 and the artificial structure 201 can be easily distinguished from the target object 200.

### (Display of Display Unit)

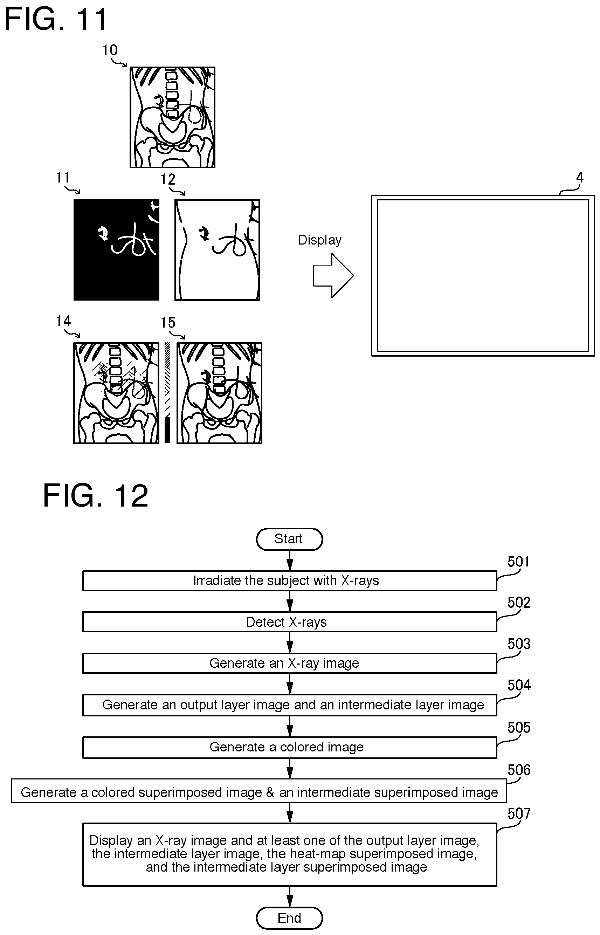

As shown in FIG. 11, in this embodiment, the image output unit 62 (control unit 6) makes the display unit 4 display and the X-ray image 10 and at least one of the output layer image 11, the intermediate layer image 12, the colored superimposed image 14, and the intermediate layer superimposed image 15, simultaneously or switchably.

For example, the image output unit 62 makes the display unit 4 display the the X-ray image 10 and the output layer image 11 simultaneously side by side. Further, for example, the image output unit 62 makes the display unit 4 display the X-ray image 10 and the output layer image 11 in a switchable manner. Further, for example, the image output unit 62 makes the display unit 4 display the X-ray image 10 and the intermediate layer image 12 side by side at the same time. Further, for example, the image output unit 62 makes the display unit 4 display the X-ray image 10 and the output layer image 12 in a switchable manner. Further, for example, the image output unit 62 makes the display unit 4 display the X-ray image 10 and the colored superimposed image 14 side by side at the same time. Further, for example, the image output unit 62 makes the display unit 4 display the X-ray image 10 and the colored superimposed image 14 in a switchable manner. Further, for example, the image output unit 62 makes the display unit 4 display the X-ray image 10 and the intermediate layer superimposed image 15 side by side at the same time. Further, for example, the image output unit 62 makes the display unit 4 display the X-ray image 10 and the intermediate layer superimposed image 15 in a switchable manner.

Further, for example, the image output unit 62 makes the display unit 4 display the X-ray image 10, the output layer image 11, and the intermediate layer image 12 side by side at the same time. Further, for example, the image output unit 62 makes the display unit 4 display the X-ray image 10, the output layer image 11, and the output layer image 12 in a switchable manner. Further, for example, the image output unit 62 makes the display unit 4 display the X-ray image 10, the output layer image 11, and the colored superimposed image 14 side by side at the same time. Further, for example, the image output unit 62 makes the display unit 4 display the X-ray image 10, the output layer image 11, and the colored superimposed image 14 in a switchable manner. Further, for example, the image output unit 62 makes the display unit 4 display the X-ray image 10, the output layer image 11, and the intermediate layer superimposed image 15 side by side at the same time. Further, for example, the image output unit 62 makes the display unit 4 display the X-ray image 10, the output layer image 11, and the intermediate layer superimposed image 15 in a switchable manner.

Further, for example, the image output unit 62 makes the display unit 4 display the X-ray image 10, the intermediate layer image 12, and the colored superimposed image 14 side by side at the same time. Further, for example, the image output unit 62 makes the display unit 4 display the X-ray image 10, the intermediate layer image 12, and the colored superimposed image 14 in a switchable manner. Further, for example, the image output unit 62 makes the display unit 4 display the X-ray image 10, the intermediate layer image 12, and the intermediate layer superimposed image 15 side by side at the same time. Further, for example, the image output unit 62 makes the display unit 4 display the X-ray image 10, the intermediate layer image 12, and the intermediate layer superimposed image 15 in a switchable manner. Further, for example, the image output unit 62 makes the display unit 4 display the X-ray image 10, the colored superimposed image 14, and the intermediate layer superimposed image 15 side by side at the same time. Further, for example, the image output unit 62 makes the display unit 4 display the X-ray image 10, the colored superimposed image 14, and the intermediate layer superimposed image 15 in a switchable manner.

Further, for example, the image output unit 62 makes the display unit 4 display the X-ray image 10, the output layer image 11, the intermediate layer image 12, and the colored superimposed image 14 side by side at the same time. Further, for example, the image output unit 62 makes the display unit 4 display the X-ray image 10, the output layer image 11, the intermediate layer image 12, and the colored superimposed image 14 in a switchable manner. Further, for example, the image output unit 62 makes the display unit 4 display the X-ray image 10, the output layer image 11, the intermediate layer image 12, and the intermediate layer superimposed image 15 side by side at the same time. Further, for example, the image output unit 62 makes the display unit 4 display the X-ray image 10, the output layer image 11, the intermediate layer image 12, and the intermediate layer superimposed image 15 in a switchable manner.

Further, for example, the image output unit 62 makes the display unit 4 display the X-ray image 10, the output layer image 11, the colored superimposed image 14, and the intermediate layer superimposed image 15 side by side at the same time. Further, for example, the image output unit 62 makes the display unit 4 display the X-ray image 10, the output layer image 11, the colored superimposed image 14, and the intermediate layer superimposed image 15 in a switchable manner. Further, for example, the image output unit 62 makes the display unit 4 display the X-ray image 10, the intermediate layer image 12, the colored superimposed image 14, and the intermediate layer superimposed image 15 side by side at the same time. Further, for example, the image output unit 62 makes the display unit 4 display the X-ray image 10, the intermediate layer image 12, the colored superimposed image 14, and the intermediate layer superimposed image 15 in a switchable manner.

Further, for example, the image output unit 62 makes the display unit 4 display the X-ray image 10, the output layer image 11, the colored superimposed image 14, and the intermediate layer superimposed image 15 side by side at the same time. Further, for example, the image output unit 62 makes the display unit 4 display the X-ray image 10, the output layer image 11, the intermediate layer image 12, the colored superimposed image 14, and the intermediate layer superimposed image 15 in a switchable manner. Note that the switching operation of each image on the display unit 4 can be performed, for example, by the input operation on the touch panel of the display unit 4.

### (Image Processing Method by This Embodiment)

Next, referring to FIG. 12, the control processing flow related to the image processing method according to this embodiment will be described. Step 501 to Step 503 show the control processing by the X-ray image generation unit 3, and Step 504 to Step 507 show the control processing by the control unit 6.

First, in Step 501, the subject 101 is irradiated with X-rays to identify the target object 200 left behind in the body of the subject 101 after surgical operation. Next, in Step 502, the irradiated X-rays are detected. Next, in Step 503, an X-ray image 10 is generated based on the detected X-ray detection signal.

Next, in Step 504, the X-ray image 10 is input to the trained model 52 to generate the output layer image 11 and the intermediate layer image 12. Next, in Step 505, a colored image 13 is generated based on the generated output layer image 11. Specifically, the portion in the output layer image 11 corresponding to the target object 200 is colored, thereby producing the colored image 13.

Next, in Step 506, the colored image 13 is superimposed on the X-ray image 10 to thereby produce a colored superimposed image 14. Further, the intermediate layer image 12 is superimposed on the X-ray image 10 to thereby produce the intermediate layer superimposed image 15. Next, in Step 507, the X-ray image 10 and at least one of the output layer image 11, the intermediate layer image 12, the colored superimposed image 14, and the intermediate layer superimposed image 15 are displayed on the display unit 4 simultaneously or switchably.

### (Effects of This Embodiment)

In this embodiment, the following effects can be obtained.

In the X-ray imaging apparatus 100 according to this embodiment, as described above, at least one of the output image (11, 12) of the trained model 52 in which the position of the target object 200 is emphasize and the composite image (14, 15) generated so that the position of the target object 200 is emphasized based on the output image (11, 12) and the X-ray image 10 is generated as an enhanced image, based on the trained model 52 that detects the region of the target object 200 in the body of the subject 101 in the X-ray image 10 when the X-ray image 10 is input. Then, the X-ray image 10 and at least one of the output image (11, 12) and the composite image (14, 15) are caused to be displayed on the display unit 4 simultaneously or switchably. With this, at least one of the output image (11, 12) and the composite image (14, 15) is generated as an enhanced image in which the position image of the target object 200 is emphasized, based on the trained model 52 that directly detects the region of the target object 200. Therefore, unlike the case in which an enhanced image in which both the target object and the human body structure, such as, e.g., a bone of the subject 101, are emphasized by edge enhancement processing, it is possible to suppress that the human body structure, such as, e.g., a bone of the subject 101, is emphasized in the enhanced image. As a result, when emphasizing the target object 200 included in the X-ray image 10 showing the subject 101, it is possible to easily confirm the target object 200 included in the X-ray image 10. Further, the X-ray image and at least one of the output image (11, 12) and the composite image (14, 15) as an enhanced image are caused to be displayed on the display unit 4 simultaneously or separately. Therefore, it is possible to easily confirm the target object 200 included in the X-ray image 10 by comparing the enhanced image with the X-ray image 10. Further, the final determination of the presence or absence of the target object 200 in the body of the subject 101 by a doctor, etc., is performed based on the X-ray image 10. Therefore, it is very effective that the target object 200 included in the X-ray image 10 can be easily confirmed by comparing the X-ray image 10 with the enhanced image.

Here, in the case of emphasizing the target object 200 in the X-ray image 10 showing the subject 101 by performing image processing using a trained model, it is conceivable to generate a trained model that removes the target object 200 from the X-ray image 10, generate a removed image in which the target object 200 has been removed from the X-ray image 10 using the generated trained model, and generate an enhanced image by the difference between the X-ray image 10 and the removed image. However, in the case where an enhanced image is generated by the difference between the X-ray image 10 and the removed image, there is a case in which not only the target object 200 but also the structure of the subject 101 similar to the target object 200 are emphasized in the enhanced image due to the removal of the structure of the subject 101 (such as pelvis and femur) that are similar to the target object 200 in the removed image. Therefore, in the case of performing image processing using a trained model that removes the target object 200 from the X-ray image 10, the visibility of the target object 200 in the enhanced image deteriorates, making it difficult to identify the target object 200 in the X-ray image 10.

Therefore, in the X-ray imaging apparatus 100 in this embodiment, as described above, at least one of the output image (11, 12) of the trained model 52 in which the position of the target object 200 is emphasized and the composite image (14, 15) generated so that the position of the target object 200 is emphasized based on the output image (11, 12) and the X-ray image 10 is generated as an enhanced image, based on the trained model 52 that detects the region of the target object 200 in the body of the subject 101 in the X-ray image 10 when the X-ray image 10 is input. Then, the X-ray image 10 and at least one of the output image (11, 12) and the composite image (14, 15) are caused to be displayed on the display unit 4 simultaneously or switchably. With this, at least one of the output image (11, 12) and the composite image (14, 15) is generated as an enhanced image in which the position of the target object 200 is emphasized, based on the trained model 52 that directly detects the region of the target object 200. Therefore, unlike the case in which a removed image is generated by a trained model that removes the target object 200 from the X-ray image 10, and an enhanced image is generated by the difference between the X-ray image 10 and the removed image, it is possible to suppress that the structure of the subject 101 similar to the target object 200 is emphasized in the enhanced image. As a result, even in the case of emphasizing the target object 200 in the X-ray image 10 showing the subject 101 by performing image processing using a learned model, the target object 200 in the X-ray image 10 can be easily confirmed.

Further, in the above-described embodiment, the following further effects can be obtained by configuring as follows.

That is, in this embodiment, as described above, the enhanced image generation unit 61 is configured to generate, as a composite image, the superimposed image (14, 15) in which the output image (12) or the image (13) generated based on the output image (11) is superimposed on the X-ray image 10. The image output unit 62 is configured to make the display unit 4 display the superimposed image (14, 15) and the X-ray image 10 simultaneously or switchably. By configuring as described above, the target object 200 included in the X-ray image 10 can be confirmed by comparing the superimposed images (14, 15) with the superimposed image (14, 15), which are images whose positional relation can be easily grasped. Therefore, it is possible to more easily confirm the target object 200 in the X-ray image 10.

Further, in this embodiment, as described above, the enhanced image generation unit 61 is configured to color the portion in the output image (11) corresponding to the target object 200 based on the output image (11) to generate the colored image 13 and also generate the superimposed image (14) in which the generated colored image 13 is superimposed on the X-ray image 10. The image output unit 62 is configured to make the display unit 4 display the superimposed image (14) in which the colored image 13 is superimposed on the X-ray image 10 and the X-ray image 10 simultaneously or switchably. By configuring as described above, the target object 200 in the X-ray image 10 can be identified by comparing the superimposed image (14) in which the colored image 13 is superimposed on the X-ray image 10 with the X-ray image 10. Therefore, the position of the target object 200 included in the X-ray image 10 can be easily and intuitively grasped based on the color of the superimposed image (14).

Further, in this embodiment, as described above, the enhanced image generation unit 61 is configured to identify the linear structure of the target object 200 in the output image (11) based on the output image (11), obtain the density in the predetermined region including the site where the linear structure of the identified target object 200 is located, and generate the colored image 13 as a heat map image colored to vary according to the density. The image output unit 62 is configured to make the display unit 4 display the superimposed image (14) in which the colored image 13 as the heat map image is superimposed and the X-ray image 10 simultaneously or switchably. By configuring as described above, the superimposed image (14) is colored according to the density in the predetermined region including the site where the linear structure of the target object 200 is located, so that it is possible to generate the superimposed image (14) in which the high-density portions are further emphasized. Further, in the output image (11) generated such that the position of the target object 200 is emphasized, the density of the linear structure is higher at the portion corresponding to the target object 200, so that the portion corresponding to the target object 200 in the superimposed image (14) can be further emphasized and colored. The target object 200 included in the X-ray image 10 can be identified by comparing the superimposed image (14) in which the colored superimposed image (13) as a heat map image in which the portion corresponding to the target object 200 is further emphasized with the X-ray image 10. Therefore, the position of the target object 200 in the X-ray image 10 can be grasped more intuitively and more easily based on the color of the superimposed image (14) in which the colored superimposed image (13) as a heat map image is superimposed.

In this embodiment, as described above, the enhanced image generation unit 61 is configured to generate the superimposed image (15) in which the intermediate layer image 12 output from the intermediate layer 52b of the trained model 52 and emphasized in the target object 200 in the X-ray image 10 is superimposed on the X-ray image 10. The image output unit 62 is configured to make the display unit 4 display the superimposed image (15) in which the intermediate layer image 12 is superimposed on the X-ray image 10 and the X-ray image 10 simultaneously or switchably. By configuring as described above, the target object 200 included in the X-ray image 10 can be identified by comparing the superimposed image (15) in which the intermediate layer image 12 capable of easily grasping the shape of the target object 200 is superimposed on with the X-ray image 10. Therefore, the target object 200 included in the X-ray image 10 can be more easily grasped based on the shape and the position of the target object 200 in the superimposed image (15).

Further, in this embodiment, as described above, the enhanced image generation unit 61 is configured to generate the output layer image 11, which is output from the output layer 52c of the trained model 52 and represents the region of the target object 200 in the X-ray image 10, and the intermediate layer image 12, which is output from the intermediate layer 52b of the trained model 52 and emphasizes the target object 200 in the X-ray image 10, as the output image. The image output unit 62 is configured to make the display unit 4 display the X-ray image 10 and at least one of the output layer image 11 and the intermediate layer image 12 simultaneously or switchably. By configuring as described above, it is possible to identify the target object 200 included in the X-ray image 10 by comparing at least one of the output layer image 11 and the intermediate layer image 12 capable of easily grasping the shape of the target object 200 with the X-ray image 10. Therefore, the target object 200 included in the X-ray image 10 can be identified more easily.

### <Modifications>

Note that the embodiments disclosed above should be considered illustrative and not restrictive in all respects. It should be noted that the scope of the invention is indicated by claims and is intended to include all modifications (modified examples) within the meaning and scope of the claims.

That is, in the above-described embodiment, an example is shown in which the X-ray imaging apparatus 100 is an X-ray imaging apparatus for rounds, but the present invention is not limited thereto. For example, the X-ray imaging apparatus 100 may be a general X-ray imaging apparatus installed in an X-ray imaging room.

Further, in the above-described embodiment, an example is shown in which the enhanced image generation unit 61 (control unit 6) is configured to generate the output layer image 11, the intermediate layer image 12, the colored superimposed image 14, and the intermediate layer superimposed image 15, but the present invention is not limited thereto. For example, the enhanced image generation unit 61 may generate at least one of the output layer image 11, the intermediate layer image 12, the colored superimposed image 14, and the intermediate layer superimposed image 15.

Further, in the above-described embodiment, an example is shown in which the enhanced image generation unit 61 (control unit 6) is configured to generate the colored image 13 based on the output layer image 11, but the present invention is not limited thereto. For example, the colored image 13 may be generated based on the intermediate layer image 12.

Further, in the above-described embodiment, an example is shown in which the enhanced image generation unit 61 (control unit 6) is configured to identify the linear structure of the target object 200 in the output layer image 11 and color it based on the identified linear structure to thereby generate the colored image 13, but the present invention is not limited thereto. For example, the colored image 13 may be generated not based on a pattern recognition of the linear structure (shape) but based on the pixel value of the output layer image 11. For example, the colored image 13 may be colored based on the pixel values of the output layer image 11.

Further, in the above-described embodiment, an example is shown in which the enhanced image generation unit 61 (control unit 6) is configured to generate the colored image 13 as a heat map image colored to vary according to the density in a predetermined region including the site where the linear structure of the target object 200 is located, but the present invention is not limited thereto. For example, not by changing colors but by setting a threshold to the density (detection value) of the linear structure, the colored image 13 in which the portion (region) larger in density than the threshold may be generated.

Further, in the above-described embodiment, an example is shown in which the enhanced image generation unit 61 (control unit 6) is configured to generate the intermediate layer superimposed image 15 in which the intermediate layer image 12 is superimposed on the X-ray image 10, but the present invention is not limited thereto. For example, an output layer superimposed image in which the output layer image 11 is superimposed on the X-ray image 10 may be generated.

Further, in the above-described embodiment, an example is shown in which the target object 200 includes surgical operation gauze, suture needles, and forceps, but the present invention is not limited thereto. For example, the target object 200 may include bolts, surgical operation wires, and surgical operation clips.

Further, in the above-described embodiment, an example is shown in which the X-ray imaging apparatus 100 is equipped with the display unit 4 that displays the image output by the image output unit 62 (control unit 6), but the present invention is not limited thereto. For example, an image output by the image output unit 62, such as, e.g., the X-ray image 10, the output layer image 11, the intermediate layer image 12, the colored superimposed image 14, and the intermediate layer superimposed image 15, may be displayed on an external display device provided separately from the X-ray imaging apparatus 100.

Further, in the above-described embodiment, an example is shown in which the X-ray imaging apparatus 100 is equipped with the control unit 6 as an image processing apparatus. For example, the generation of the enhanced image, such as, e.g., the output layer image 11, the intermediate layer image 12, the colored superimposed image 14, and the intermediate layer superimposed image 15, may be performed by an image processing apparatus provided separately from the X-ray imaging apparatus 100.

Further, in the above-described embodiment, an example is shown in which the enhanced image generation unit 61 (control unit 6) is configured to generate the colored image 13 so that the density (detection value) of the linear structure (shape) can be identified by varying the hue, but the present invention is not limited thereto. For example, the colored image 13 may be generated by varying the luminance of a single hue (e.g., red) so that the density ("detection value") of the linear structure (shape) can be distinguished. In other words, the colored image 13 may be generated so that the luminance increases when the detection value is large and decreases when the detection value is small.

Further, in the above-described embodiment, an example is shown in which the control processing for generating the X-ray image 10 and the control processing for generating the enhanced image are performed by the X-ray image generation unit 3 and the control unit 6, which are configured as separate hardware, but the present invention is not limited thereto. For example, the generation of the X-ray image 10 and the generation of the enhanced image may be performed by a single common control unit (hardware).

Further, in the above-described embodiment, an example is shown in which the enhanced image generation unit 61 and the image output unit 62 are each configured as a functional block (software) in a single hardware (control unit 6), but the present invention is not limited thereto. For example, the enhanced image generation unit 61 and the image output unit 62 each may be composed of separate hardware (operation circuit).

Further, in the above-described embodiment, an example is shown in which the trained model 52 is generated by a training device 300 provided separately from the X-ray imaging apparatus 100, but the present invention is not limited thereto. For example, the trained model 52 may be generated by the X-ray imaging apparatus 100.

Further, in the above-described embodiment, an example is shown in which the trained model 52 is generated based on a U-Net, which is one type of a fully convolutional network (Fully Convolution Network: FCN), but the present invention is not limited thereto. For example, the trained model 52 may be generated based on a CNN (Convolutional Neural Network) including a fully connected layer. Further, the trained model 52 may be generated based on an Encoder-Decoder model other than a U-Net, such as, e.g., a SegNet or a PSPNet.

### Description of Reference Symbols

- 1:: X-ray irradiation unit
- 2:: X-ray detection unit
- 3:: X-ray image generation unit
- 4:: Display unit
- 6:: Control unit (Image processing apparatus, Computer)
- 10:: X-ray image
- 11:: Output layer image (Enhanced image, Output image)
- 12:: Intermediate layer image (Enhanced image, Output image)
- 13:: Colored image
- 14:: Colored superimposed image (Enhanced image, Composite image, Superimposed image)
- 15:: Intermediate layer superimposed image (Enhanced image, Composite image, Superimposed image)
- 51:: Image processing program
- 52:: Trained model
- 52b:: Intermediate layer
- 52c:: Output layer
- 61:: Enhanced image generation unit
- 62:: Image output unit
- 100:: X-ray imaging apparatus
- 101:: Subject
- 200:: Target object

## Claims

1. An X-ray imaging apparatus (100) comprising:
an X-ray irradiation unit (1) configured to irradiate a subject (101) with X-rays;
an X-ray detection unit (2) configured to detect X-rays emitted from the X-ray irradiation unit (1);
an X-ray image generation unit (3) configured to generate an X-ray image (10) based on a detection signal of X-rays detected by the X-ray detection unit (2); and
a control unit (6),
wherein the control unit (6) includes:
an enhanced image generation unit (61) configured to generate, as an enhanced image (11, 12, 14, 15), at least one of an output image (11, 12) of a trained model (52) in which a position of a retained foreign object (200) is emphasized and a composite image (14, 15) generated such that the position of the retained foreign object (200) is emphasized based on the output image (11, 12) and the X-ray image (10), the output image (11, 12) is generated based on the trained model (52) that detects a region of the retained foreign object (200) in a body of the subject (101) in the X-ray image (10) when the X-ray image (10) generated by the X-ray image generation unit (3) is input;
**characterised in that**
an image output unit (62) configured to make a display unit (4) display the X-ray image (10) and at least one of the output image (11, 12) and the composite image (14, 15) simultaneously or switchably, the output image (11, 12) and the composite image (14, 15) being generated by the enhanced image generation unit (61),
wherein the trained model (52) includes an intermediate (52b) layer having a down-sampling unit that acquires feature components from the X-ray image (10) as an input image of the trained model (52) while reducing a size of the X-ray image (10), and an up-sampling unit that restores an image including the feature components reduced in size by the down-sampling unit to a size of the X-ray image (10).

2. The X-ray imaging apparatus (100) as recited in claim 1,
wherein the enhanced image generation unit (61) is configured to generate, as the composite image (14, 15), a superimposed image (14, 15) in which the output image (11) or an image (13) generated based on the output image (12) is superimposed on the X-ray image (10), and
wherein the image output unit (62) is configured to make the display unit (4) display the superimposed image (14, 15) and the X-ray image (10) simultaneously or switchably.

3. The X-ray imaging apparatus (100) as recited in claim 2,
wherein the enhanced image generation unit (61) is configured to generate a colored image (13) by coloring a portion in the output image (11) corresponding to the retained foreign object (200) based on the output image (11) and generate the superimposed image (14) in which the generated colored image (13) is superimposed on the X-ray image (10), and
wherein the image output unit (62) is configured to make the display unit (4) display the superimposed image (14) in which the colored image (13) is superimposed on the X-ray image (10) and the X-ray image (10) simultaneously or switchably.

4. The X-ray imaging apparatus (100) as recited in claim 3,
wherein the enhanced image generation unit (61) is configured to identify a linear structure of the retained foreign object (200) in the output image (11) based on the output image (11), acquire a density in a predetermined region including a site where the identified linear structure of the retained foreign object (200) is located, and generate the colored image (13) as a heat map image colored to vary according to the density, and
wherein the image output unit (62) is configured to make the display unit (4) display the superimposed image (14) in which the colored image (13) as the heat map image is superimposed and the X-ray image (10) simultaneously or switchably.

5. The X-ray imaging apparatus (100) as recited in claim 2,
wherein the enhanced image generation unit (61) is configured to generate the superimposed image (15) in which an intermediate layer image (12) as the output image (12) which is output from an intermediate layer (52b) of the trained model (52) and emphasized in the retained foreign object (200) in the X-ray image (10) is superimposed on the X-ray image (10),
wherein the image output unit (62) is configured to make the display unit (4) display the superimposed image (15) in which the intermediate layer image (12) is superimposed on the X-ray image (10) and the X-ray image (10) simultaneously or switchably, and
wherein the intermediate layer (52b) has a down-sampling unit that acquires feature components from the X-ray image (10) as a input image of the trained model (52) while reducing a size of the X-ray image (10), and an up-sampling unit that restores an image including the feature components reduced in size by the down-sampling unit to a size of the X-ray image (10).

6. The X-ray imaging apparatus (100) as recited in claim 1,
wherein the enhanced image generation unit (61) is configured to generate, as the output image (11, 12), at least one of an output layer image (11) that is output from an output layer (52c) of the trained model (52) and represents the region of the retained foreign object (200) in the X-ray image (10) and an intermediate layer image (12) that is output from an intermediate layer (52b) of the trained model (52) and emphasized in the retained foreign object (200) in the X-ray image (10),
wherein the image output unit (62) is configured to make the display unit (4) display the X-ray image (10) and at least one of the output layer image (11) and the intermediate layer image (12) simultaneously or switchably, and
wherein the intermediate layer (52b) has a down-sampling unit that acquires feature components from the X-ray image (10) as a input image of the trained model (52) while reducing a size of the X-ray image (10), and an up-sampling unit that restores an image including the feature components reduced in size by the down-sampling unit to a size of the X-ray image (10).

7. An image processing program configured to make a computer execute:
processing for generating, as an enhanced image (11, 12, 14, 15), at least one of an output image (11, 12) of a trained model (52) in which a position of a retained foreign object (200) is emphasized and a composite image (14, 15) generated so that the position of the retained foreign object (200) is emphasized based on the output image (11, 12) and an X-ray image (10), the output image (11, 12) is generated based on the trained model (52) that detects a region of the retained foreign object (200) in a body of a subject (101) in the X-ray image (10) when the X-ray image (10) generated based on a detection signal of X-rays emitted to the subject (101) is input;
**characterised in that**
processing for making a display unit (4) display the X-ray image (10) and at least one of the output image (11, 12) and the composite image (14, 15) simultaneously or switchably,
wherein the trained model (52) includes an intermediate (52b) layer having a down-sampling unit that acquires feature components from the X-ray image (10) as an input image of the trained model (52) while reducing a size of the X-ray image (10), and an up-sampling unit that restores an image including the feature components reduced in size by the down-sampling unit to a size of the X-ray image (10).

## Patentansprüche

1. Röntgenbildgebungseinrichtung (100), umfassend:
eine Röntgenbestrahlungseinheit (1), die konfiguriert ist, um ein Subjekt (101) mit Röntgenstrahlen zu bestrahlen;
ein Röntgendetektionseinheit (2), die konfiguriert ist, um die Röntgenstrahlen, die von der Röntgendetektionseinheit (1) emittiert werden, zu detektieren;
eine Röntgenbilderzeugungseinheit (3), die konfiguriert ist, um ein Röntgenbild (10) basierend auf einem Detektionssignal von Röntgenstrahlen, die durch die Röntgendetektionseinheit (2) detektiert werden, zu erzeugen; und
eine Steuereinheit (6),
wobei die Steuereinheit (6) einschließt:
eine Einheit (61) zur Erzeugung eines verbesserten Bildes, die konfiguriert ist, um als ein verbessertes Bild (11, 12, 14, 15) mindestens eines von einem Ausgabebild (11, 12) eines trainierten Modells (52), in dem eine Position eines zurückgebliebenen Fremdkörpers (200) hervorgehoben ist, und einem zusammengesetzten Bild (14, 15), das derart erzeugt wird, dass die Position des zurückgebliebenen Fremdkörpers (200) basierend auf dem Ausgabebild (11, 12) und dem Röntgenbild (10) hervorgehoben ist, zu erzeugen, wobei das Ausgabebild (11, 12) basierend auf dem trainierten Modell (52) erzeugt wird, das einen Bereich des zurückgebliebenen Fremdkörpers (200) in einem Körper des Subjekts (101) in dem Röntgenbild (10) detektiert, wenn das Röntgenbild (10) durch die Röntgenbilderzeugungseinheit (3) erzeugt wird, eingegeben wird;
**dadurch gekennzeichnet, dass**
eine Bildausgabeeinheit (62) konfiguriert ist, um eine Anzeigeeinheit (4) zu veranlassen, das Röntgenbild (10) und mindestens eines von dem Ausgabebild (11, 12) und dem zusammengesetzten Bild (14, 15) gleichzeitig oder umschaltbar anzuzeigen, wobei das Ausgabebild (11, 12) und das zusammengesetzte Bild (14, 15) durch die Einheit (61) zur Erzeugung eines verbesserten Bildes erzeugt werden,
wobei das trainierte Modell (52) eine Zwischen(52b)-Schicht einschließt, die eine Downsampling-Einheit, die Merkmalskomponenten aus dem Röntgenbild (10) als ein Eingabebild des trainierten Modells (52) während des Reduzierens einer Größe des Röntgenbildes (10) erfasst, und eine Upsampling-Einheit, die ein Bild wiederherstellt, einschließlich der Merkmalskomponenten, die in Größe durch die Downsampling-Einheit auf eine Größe des Röntgenbildes (10) reduziert werden, aufweist.

2. Röntgenbildgebungseinrichtung (100) nach Anspruch 1,
wobei die Einheit (61) zur Erzeugung eines verbesserten Bildes konfiguriert ist, um als das zusammengesetzte Bild (14, 15) ein überlagertes Bild (14, 15) zu erzeugen, in dem das Ausgabebild (11) oder ein basierend auf dem Ausgabebild (12) erzeugtes Bild (13) dem Röntgenbild (10) überlagert ist, und
wobei die Bildausgabeeinheit (62) konfiguriert ist, um die Anzeigeeinheit (4) zu veranlassen, das überlagerte Bild (14, 15) und das Röntgenbild (10) gleichzeitig oder umschaltbar anzuzeigen.

3. Röntgenbildgebungseinrichtung (100) nach Anspruch 2,
wobei die Einheit (61) zur Erzeugung eines verbesserten Bildes konfiguriert ist, um ein farbiges Bild (13) durch Färben eines Abschnitts in dem Ausgabebild (11), der dem zurückgebliebenen Fremdkörper (200) entspricht, basierend auf dem Ausgabebild (11) zu erzeugen und das überlagerte Bild (14) zu erzeugen, in dem das erzeugte farbige Bild (13) auf dem Röntgenbild (10) überlagert ist, und
wobei die Bildausgabeeinheit (62) konfiguriert ist, um die Anzeigeeinheit (4) zu veranlassen, das überlagerte Bild (14), in dem das farbige Bild (13) auf dem Röntgenbild (10) überlagert ist, und das Röntgenbild (10) gleichzeitig oder umschaltbar anzuzeigen.

4. Röntgenbildgebungseinrichtung (100) nach Anspruch 3,
wobei die Einheit (61) zur Erzeugung eines verbesserten Bildes konfiguriert ist, um eine lineare Struktur des zurückgebliebenen Fremdkörpers (200) in dem Ausgabebild (11) basierend auf dem Ausgabebild (11) zu identifizieren, eine Dichte in eines vorbestimmten Bereichs zu erfassen, einschließlich einer Stelle, an der sich die identifizierte lineare Struktur des zurückgebliebenen Fremdkörpers (200) befindet, und das farbige Bild (13) als ein Heatmap-Bild zu erzeugen, das gefärbt ist, um gemäß der Dichte zu variieren, und
wobei die Bildausgabeeinheit (62) konfiguriert ist, um die Anzeigeeinheit (4) zu veranlassen, das überlagerte Bild (14), in dem das farbige Bild (13) als das Heatmap-Bild überlagert ist, und das Röntgenbild (10) gleichzeitig oder umschaltbar anzuzeigen.

5. Röntgenbildgebungseinrichtung (100) nach Anspruch 2,
wobei die Einheit (61) zur Erzeugung eines verbesserten Bildes konfiguriert ist, um das überlagerte Bild (15) zu erzeugen, in dem ein Zwischenschichtbild (12) als das Ausgabebild (12), das von einer Zwischenschicht (52b) des trainierten Modells (52) ausgegeben wird und in dem zurückgehaltenen Fremdkörper (200) in dem Röntgenbild (10) hervorgehoben ist, auf dem Röntgenbild (10) überlagert wird,
wobei die Bildausgabeeinheit (62) konfiguriert ist, um die Anzeigeeinheit (4) zu veranlassen, das überlagerte Bild (15), in dem das Zwischenschichtbild (12) auf dem Röntgenbild (10) überlagert ist, und das Röntgenbild (10) gleichzeitig oder umschaltbar anzuzeigen und
wobei die Zwischenschicht (52b) eine Downsampling-Einheit, die Merkmalskomponenten aus dem Röntgenbild (10) als ein Eingabebild des trainierten Modells (52) während des Reduzierens einer Größe des Röntgenbildes (10) erfasst, und eine Upsampling-Einheit, die ein Bild wiederherstellt, einschließlich der Merkmalskomponenten, die in Größe durch die Downsampling-Einheit auf eine Größe des Röntgenbildes (10) reduziert werden, aufweist.

6. Röntgenbildgebungseinrichtung (100) nach Anspruch 1,
wobei die Einheit (61) zur Erzeugung eines verbesserten Bildes konfiguriert ist, um als das Ausgabebild (11, 12) mindestens eines von einem Ausgabeschichtbild (11), das von einer Ausgabeschicht (52c) des trainierten Modells (52) ausgegeben wird und den Bereich des zurückgehaltenen Fremdkörpers (200) in dem Röntgenbild (10) darstellt, und einem Zwischenschichtbild (12), das von einer Zwischenschicht (52b) des trainierten Modells (52) ausgegeben wird und bei dem der zurückgehaltene Fremdkörper (200) in dem Röntgenbild (10) hervorgehoben ist, zu erzeugen,
wobei die Bildausgabeeinheit (62) konfiguriert ist, um die Anzeigeeinheit (4) zu veranlassen, das Röntgenbild (10) und mindestens eines von dem Ausgabeschichtbild (11) und dem Zwischenschichtbild (12) gleichzeitig oder umschaltbar anzuzeigen, und
wobei die Zwischenschicht (52b) eine Downsampling-Einheit, die Merkmalskomponenten aus dem Röntgenbild (10) als ein Eingabebild des trainierten Modells (52) während des Reduzierens einer Größe des Röntgenbildes (10) erfasst, und eine Upsampling-Einheit, die ein Bild wiederherstellt, einschließlich der Merkmalskomponenten, die in Größe durch die Downsampling-Einheit auf eine Größe des Röntgenbildes (10) reduziert werden, aufweist.

7. Bildverarbeitungsprogramm, das konfiguriert ist, um einen Computer zu veranlassen, Folgendes auszuführen:
Verarbeiten zum Erzeugen, als ein verbessertes Bild (11, 12, 14, 15), mindestens eines von einem Ausgabebild (11, 12) eines trainierten Modells (52), in dem eine Position eines zurückgebliebenen Fremdkörpers (200) hervorgehoben ist, und einem zusammengesetzten Bild (14, 15), das erzeugt wird, sodass die Position des zurückgebliebenen Fremdkörpers (200) basierend auf dem Ausgabebild (11, 12) und einem Röntgenbild (10) hervorgehoben ist, wobei das Ausgabebild (11, 12) basierend auf dem trainierten Modell (52) erzeugt wird, das einen Bereich des zurückgebliebenen Fremdkörpers (200) in dem Körper eines Subjekts (101) in dem Röntgenbild (10) detektiert, wenn das Röntgenbild (10), das basierend auf einem Detektionssignal von auf das Subjekt (101) emittierten Röntgenstrahlen erzeugt wird, eingegeben wird;
**dadurch gekennzeichnet, dass**
das Verarbeiten zum Veranlassen einer Anzeigeeinheit (4) das Röntgenbild (10) und mindestens eines von dem Ausgabebild (11, 12) und dem zusammengesetzten Bild (14, 15) gleichzeitig oder umschaltbar anzuzeigen,
wobei das trainierte Modell (52) eine Zwischen(52b)-Schicht einschließt, die eine Downsampling-Einheit, die Merkmalskomponenten aus dem Röntgenbild (10) als ein Eingabebild des trainierten Modells (52) während des Reduzierens einer Größe des Röntgenbildes (10) erfasst, und eine Upsampling-Einheit, die ein Bild wiederherstellt, einschließlich der Merkmalskomponenten, die in Größe durch die Downsampling-Einheit auf eine Größe des Röntgenbildes (10) reduziert werden, aufweist.

## Revendications

1. Appareil d'imagerie par rayons X (100) comprenant :
une unité d'irradiation par rayons X (1) configurée pour irradier un sujet (101) avec des rayons X ;
une unité de détection de rayons X (2) configurée pour détecter des rayons X émis à partir de l'unité d'irradiation par rayons X (1) ;
une unité de génération d'image radiologique (3) configurée pour générer une image radiologique (10) sur la base d'un signal de détection de rayons X détectés par l'unité de détection de rayons X (2) ; et
une unité de commande (6),
dans lequel l'unité de commande (6) comporte :
une unité de génération d'image améliorée (61) configurée pour générer, en tant qu'image améliorée (11, 12, 14, 15), au moins l'une parmi une image de sortie (11, 12) d'un modèle entraîné (52) dans laquelle une position d'un objet étranger retenu (200) est accentuée et une image composite (14, 15) générée de telle sorte que la position du corps étranger retenu (200) est accentuée en fonction de l'image de sortie (11, 12) et l'image radiologique (10), l'image de sortie (11, 12) est générée en fonction du modèle entraîné (52) qui détecte une région du corps étranger retenu (200) dans un corps du sujet (101) dans l'image radiologique (10) lorsque l'image radiologique (10) générée par l'unité de génération d'images radiologiques (3) est entrée ;
**caractérisé en ce que**
une unité de sortie d'image (62) configurée pour qu'une unité d'affichage (4) affiche l'image radiologique (10) et au moins l'une parmi l'image de sortie (11, 12) et l'image composite (14, 15) simultanément ou de manière commutable, l'image de sortie (11, 12) et l'image composite (14, 15) étant générées par l'unité de génération d'image améliorée (61),
dans lequel le modèle entraîné (52) comporte une couche intermédiaire (52b) ayant une unité de sous-échantillonnage qui acquiert des composants de caractéristique à partir de l'image radiologique (10) en tant qu'image d'entrée du modèle entraîné (52) tout en réduisant une taille de l'image radiologique (10), et une unité de sur-échantillonnage qui restaure une image comportant les composants de caractéristique réduits en taille par l'unité de sous-échantillonnage à une taille de l'image radiologique (10).

2. Appareil d'imagerie par rayons X (100) selon la revendication 1,
dans lequel l'unité de génération d'image améliorée (61) est configurée pour générer, en tant qu'image composite (14, 15), une image superposée (14, 15) dans laquelle l'image de sortie (11) ou une image (13) générée sur la base de l'image de sortie (12) est superposée à l'image radiologique (10), et
dans lequel l'unité de sortie d'image (62) est configurée pour que l'unité d'affichage (4) affiche l'image superposée (14, 15) et l'image radiologique (10) simultanément ou de manière commutable.

3. Appareil d'imagerie par rayons X (100) selon la revendication 2,
dans lequel l'unité de génération d'image améliorée (61) est configurée pour générer une image colorée (13) par coloration d'une partie de l'image de sortie (11) correspondant au corps étranger retenu (200) en fonction de l'image de sortie (11) et générer l'image superposée (14) dans laquelle l'image colorée (13) générée est superposée à l'image radiologique (10), et
dans lequel l'unité de sortie d'image (62) est configurée pour que l'unité d'affichage (4) affiche l'image superposée (14) dans laquelle l'image colorée (13) est superposée à l'image radiologique (10) et à l'image radiologique (10) simultanément ou de manière commutable.

4. Appareil d'imagerie par rayons X (100) selon la revendication 3,
dans lequel l'unité de génération d'image améliorée (61) est configurée pour identifier une structure linéaire du corps étranger retenu (200) dans l'image de sortie (11) sur la base de l'image de sortie (11), acquérir une densité dans une région prédéterminée comportant un site où la structure linéaire identifiée du corps étranger retenu (200) est située, et générer l'image colorée (13) sous forme d'image de carte thermique colorée pour varier selon la densité, et
dans lequel l'unité de sortie d'image (62) est configurée pour que l'unité d'affichage (4) affiche l'image superposée (14) dans laquelle l'image colorée (13) en tant qu'image de carte thermique est superposée et l'image radiologique (10) simultanément ou de manière commutable.

5. Appareil d'imagerie par rayons X (100) selon la revendication 2,
dans lequel l'unité de génération d'images améliorées (61) est configurée pour générer l'image superposée (15) dans laquelle une image de couche intermédiaire (12) en tant qu'image de sortie (12) qui est émise à partir d'une couche intermédiaire (52b) du modèle entraîné (52) et soulignée dans le corps étranger retenu (200) dans l'image radiologique (10) est superposée à l'image radiologique (10),
dans lequel l'unité de sortie d'image (62) est configurée pour que l'unité d'affichage (4) affiche l'image superposée (15) dans laquelle l'image de couche intermédiaire (12) est superposée à l'image radiologique (10) et à l'image radiologique (10) simultanément ou de manière commutable, et
dans lequel la couche intermédiaire (52b) a une unité de sous-échantillonnage qui acquiert des composantes de caractéristiques à partir de l'image radiologique (10) en tant qu'image d'entrée du modèle entraîné (52) tout en réduisant une taille de l'image radiologique (10), et une unité de suréchantillonnage qui restaure une image comportant les composantes de caractéristiques réduites en taille par l'unité de sous-échantillonnage à une taille de l'image radiologique (10).

6. Appareil d'imagerie par rayons X (100) selon la revendication 1,
dans lequel l'unité de génération d'image améliorée (61) est configurée pour générer, en tant qu'image de sortie (11, 12), au moins l'une parmi une image de couche de sortie (11) qui est émise à partir d'une couche de sortie (52c) du modèle entraîné (52) et représente la région du corps étranger retenu (200) dans l'image radiologique (10) et une image de couche intermédiaire (12) qui est émise à partir d'une couche intermédiaire (52b) du modèle entraîné (52) et soulignée dans le corps étranger retenu (200) dans l'image radiologique (10),
dans lequel l'unité de sortie d'image (62) est configurée pour que l'unité d'affichage (4) affiche l'image radiologique (10) et au moins l'une parmi l'image de couche de sortie (11) et l'image de couche intermédiaire (12) simultanément ou de manière commutable, et
dans lequel la couche intermédiaire (52b) a une unité de sous-échantillonnage qui acquiert des composantes de caractéristiques à partir de l'image radiologique (10) en tant qu'image d'entrée du modèle entraîné (52) tout en réduisant une taille de l'image radiologique (10), et une unité de suréchantillonnage qui restaure une image comportant les composantes de caractéristiques réduites en taille par l'unité de sous-échantillonnage à une taille de l'image radiologique (10).

7. Programme de traitement d'images configuré pour faire exécuter par un ordinateur :
le traitement pour générer, en tant qu'image améliorée (11, 12, 14, 15), au moins l'une parmi une image de sortie (11, 12) d'un modèle entraîné (52) dans laquelle une position d'un objet étranger retenu (200) est accentuée et une image composite (14, 15) générée de sorte que la position du corps étranger retenu (200) est accentuée sur la base de l'image de sortie (11, 12) et une image radiologique (10), l'image de sortie (11, 12) est générée en fonction du modèle entraîné (52) qui détecte une région du corps étranger retenu (200) dans un corps d'un sujet (101) dans l'image radiologique (10) lorsque l'image radiologique (10) générée en fonction d'un signal de détection de rayons X émis vers le sujet (101) est entrée ;
**caractérisé en ce que**
un traitement permettant à une unité d'affichage (4) d'afficher l'image radiologique (10) et au moins l'une parmi l'image de sortie (11, 12) et l'image composite (14, 15) simultanément ou de manière commutable,
dans lequel le modèle entraîné (52) comporte une couche intermédiaire (52b) ayant une unité de sous-échantillonnage qui acquiert des composants de caractéristique à partir de l'image radiologique (10) en tant qu'image d'entrée du modèle entraîné (52) tout en réduisant une taille de l'image radiologique (10), et une unité de sur-échantillonnage qui restaure une image comportant les composants de caractéristique réduits en taille par l'unité de sous-échantillonnage à une taille de l'image radiologique (10).
